Europäisches Patentamt

European Patent Office

Office européen des brevets

(11) Veröffentlichungsnummer: **0 360 734**

**A1**

## (12) EUROPÄISCHE PATENTANMELDUNG

(21) Anmeldenummer: 89810569.7

(22) Anmeldetag: 26.07.89

(51) Int. Cl.⁵: **A 61 F 2/32**

(30) Priorität: 06.09.88 CH 3343/88

(43) Veröffentlichungstag der Anmeldung:
28.03.90 Patentblatt 90/13

(84) Benannte Vertragsstaaten: **AT DE FR GB IT**

(71) Anmelder: **GEBRÜDER SULZER
AKTIENGESELLSCHAFT
Zürcherstrasse 9
CH-8401 Winterthur (CH)**

(72) Erfinder: **Willert, Hans-Georg, Prof. Dr.-med.
Schlegelweg 9
D-3400 Göttingen (DE)**

**Koch, Rudolf
Oberdorfstrasse 229
CH 8267 Berlingen (DE)**

(54) Femurkopf-Prothese.

(57) Die Aussenfläche (16) des Halses (3) des Gelenkkopfes (1) ist an die Randfläche (15) des Schalenkörpers (7) als Gleitlagerfläche angepasst.

Bei extremen Auslenkungen des Gelenkkopfes (1) erfolgt die gegenseitige Abstützung von Kugelschale (5) und Gelenkkopf (1) dadurch über eine Linien- statt über eine Punktberührung, wodurch örtliche Ueberlastungen verhindert und Abrieb zumindest vermindert werden.

Bundesdruckerei Berlin

EP 0 360 734 A1

**Beschreibung**

## Femurkopf-Prothese

Die Erfindung betrifft eine Femurkopfprothese mit einem inneren Gelenkkopf, der über eine Konussteckverbindung fest mit einem Verankerungsschaft verbunden ist und einen halsartigen Ansatz aufweist, und mit einer beweglich auf dem inneren Gelenkkopf gelagerten, äusseren Kugelschale, die aus einer harten Aussenhaube und einem in dieser befestigten Kunststoff-Schalenkörper für die Aufnahme des inneren Gelenkkopfes besteht, wobei Kugelschale und Gelenkkopf aus einer zentralen Lage heraus, bei der die Rotationssymmetrieachse der Kugelschale und die Halsachse des Gelenkkopfes zusammenfallen, gegeneinander verschwenkbar sind.

Eine Femurkopfprothese der genannten Art ist beispielsweise aus der Druckschrift "Madreporische Prothese ohne Zement" der Firma Howmedica GmbH., Kiel/BRD, bekannt.

Derartige Prothesen werden im allgemeinen in solchen Fällen eingesetzt, in denen das Azetabulum des Beckens noch gesund ist. Die äussere Kugelschale ist dabei direkt im Azetabulum gelagert. Bei Bewegungen im "normalen" Winkelbereich des Hüftgelenks bilden bei diesen Prothesen der Gelenkkopf und die Pfannenschale im Kunststoff-Schalenkörper das Lager, in dem die Bewegung stattfindet. Die Aussenschale verharrt dabei relativ unbeweglich im Azetabulum.

Bei extrem Ausschlägen des Gelenkkopfes stossen der Prothesenhals bzw. der Hals des Gelenkkopfes an den Rand des Schalenkörpers an und veranlassen eine Bewegung zwischen Aussenschale und Azetabulum. Bei diesen Bewegungen treten Relativverschiebungen zwischen Schalenkörper und Prothesen- bzw. Gelenkkopfhals auf. Diese sind bei bisherigen Konstruktionen mit erheblicher Reibung verbunden. Darüberhinaus erfolgen bei den bekannten Konstruktionen die Auflagen von Schalenkörper und Hals nur als Punktberührungen, was zu unzulässig hohen örtlichen Belastungen des Kunststoff-Schalenkörpers und zu plastischem Fliessen des Kunststoffes führen kann.

Ferner ist aus der DE-A-36 43 815 eine Prothesenkonstruktion bekannt, bei der der auf einem Schaft befestigte Gelenkkopf in einer äusseren Kugelschale so gelagert ist, dass er zwar um die Halsachse des Gelenkkopfes reine Rotationsbewegungen ausführen, jedoch gegen die Symmetrieachse der Kugelschale nicht verschwenkt werden kann.

Aufgabe der Erfindung ist es, die Reibung und damit den Abrieb bei Prothesen der eingangs genannten Art zu verringern. Diese Aufgabe wird mit der Erfindung dadurch gelöst, dass die Aussenfläche des halsartigen Ansatzes des Gelenkkopfes als Gleitlagerfläche an die dem Verankerungsschaft zugewandte Randfläche des Schalenkörpers über mindestens 50 % der kontaktfähigen Flächenanteile beider angepasst ist.

Die Ausbildung des Gelenkkopfhalses und des Randes des Schalenkörpers als Gleitlager bewirkt, dass zwischen beiden eine Linienberührung auftritt, so dass örtliche Ueberlastungen vermieden und Abrieb vermindert werden. Die Gleiteigenschaften dieses Lagers können darüberhinaus verbessert werden, wenn mindestens die Aussenfläche eine hochglanzpolierte Gleitfläche bildet. Weiterhin lässt sich die Stabilität der Lagerung des Gelenkkopfes in der Pfannenschale des Schalenkörpers erhöhen, wenn das Kugelzentrum der Kugelschale gegenüber demjenigen des Gelenkkopfes zum Verankerungsschaft hin verschoben ist.

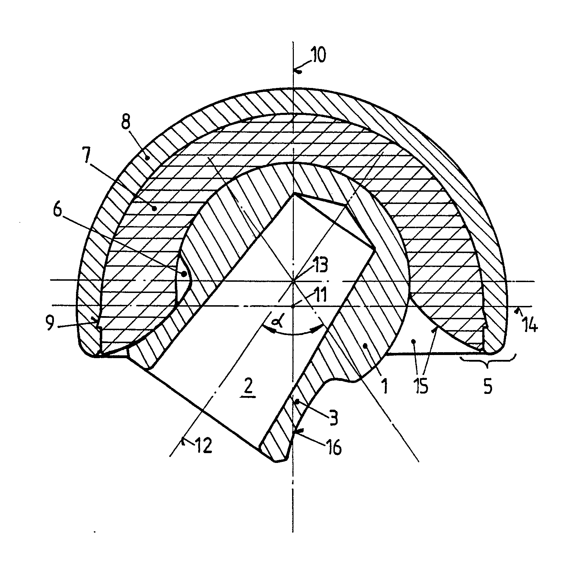

Im folgenden wird die Erfindung anhand eines Ausführungsbeispiels im Zusammenhang mit der Zeichnung näher erläutert.

Die einzige Figur zeigt einen Meridianschnitt durch den Gelenkkopf und die Kugelschale.

Der Gelenkkopf 1, der aus Metall oder Keramik bestehen kann, ist über eine Konussteckverbindung, deren Mutterkonus 2 er trägt, auf einem nicht gezeigten Zapfen eines Verankerungsschaftes befestigt. Sein eigentlicher Kugelkopf geht zu diesem Schaft hin in einen halsartigen Ansatz 3 über.

Drehbar auf dem Gelenkkopf 1 ist eine Kugelschale 5 gelagert. Bei dieser Lagerung ruht der Gelenkkopf 1 in einer Pfannenschale 6 eines Kunststoff-Schalenkörpers 7, der aussen von einer aus Metall oder Keramik hergestellten Aussenhaube 8 umgeben ist. Schalenkörper 7 und Aussenhaube 8 sind in bekannter Weise über einen Schnappverschluss 9 miteinander verbunden.

Die Rotationssymmetrieachse der Kugelschale 5 und ihr Mittelpunkt sind mit 10 und 11, die Halsachse des Gelenkkopfes 1 und dessen Mittelpunkt mit 12 und 13 bezeichnet.

Die Kugelschale 5 ist in ihrer Form, insbesondere in den von ihr über den Aequator 14 hinaus noch erfassten "geographischen Breiten" so gestaltet, dass der Gelenkkopf 1 aus einer zentralen Lage heraus, bei der die Achsen 10 und 12 zusammenfallen, in einem Kegelbereich mit einem Oeffnungswinkel $\alpha$ von 70° verschwenkbar ist.

Der dem Hals 3 des Gelenkkopfes 1 zugewandte Rand 15 des Schalenkörpers 7 und die Aussenfläche 16 des Prothesenhalses 3 sind derart einander angepasst, dass beide mindestens über den grössten Teil ihrer, möglicherweise miteinander kontaktfähigen Flächen ein Gleitlager bilden. Um die Gleitfähigkeit zu erhöhen, ist die Ausenfläche 16 mit einer hochglanzpolierten Oberfläche versehen.

Zur Vergrösserung der Stabilität der Lagerung des Gelenkkopfes 1 in der Pfannenschale 6 liegt der Mittelpunkt 11 der Kugelschale 5 gegenüber demjenigen 13 des Gelenkkopfes 1 auf der Achse 10 zum nicht gezeigten Verankerungsschaft hin verschoben. Dadurch wird erreicht, dass der Gelenkkopf 1 - wenn er bei die gezeigte Stellung übersteigenden Auslenkungen aus der Pfannenschale 6 herauszukippen beginnt - bei Nachlassen äusserer Kraft von selbst wieder in die Pfannenschale 6 zurückfällt.

**Patentansprüche**

1. Femurkopfprothese mit einem inneren Gelenkkopf, der über eine Konussteckverbindung fest mit einem Verankerungsschaft verbunden ist und einen halsartigen Ansatz aufweist, und mit einer beweglich auf dem innneren Gelenkkopf gelagerten, äusseren Kugelschale, die aus einer harten Aussenhaube und einem in dieser befestigten Kunststoff-Schalenkörper für die Aufnahme des inneren Gelenkkopfes besteht, wobei Kugelschale und Gelenkkopf aus einer zentralen Lage heraus, bei der die Rotationssymmetrieachse der Kugelschale und die Halsachse des Gelenkkopfes zusammenfallen, gegeneinander verschwenkbar sind, dadurch **gekennzeichnet** , dass die Aussenfläche (16) des halsartigen Ansatzes (3) des Gelenkkopfes (1) als Gleitlagerfläche an die dem Verankerungsschaft zugewandte Randfläche (15) des Schalenkörpers (7) über mindestens 50% der kontaktfähigen Flächenanteile beider angepasst ist.

2. Femurkopfprothese nach Anspruch 1, dadurch gekennzeichnet, dass mindestens die Aussenfläche (16) eine hochglanzpolierte Gleitfläche bildet.

3. Femurkopfprothese nach Anspruch 1 oder 2, dadurch gekennzeichnet, dass das Kugelzentrum (11) der Kugelschale (5) gegenüber demjenigen (13) des Gelenkopfes (1) zum Verankerungsschaft hin verschoben ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 524 467 (DECARLO) <br> * Figuren 5,6; Spalte 7, Zeile 55 - Spalte 8, Zeile 13 * | 1 | A 61 F 2/32 |
| Y | | 3 | |
| Y | FR-A-2 387 641 (DEZEUZE) <br> * Ansprüche; Figuren * | 3 | |
| | --- | | |
| A | FR-A-2 516 377 (BREARD) | | |
| | --- | | |
| A | FR-A-2 551 655 (BREARD) | | |
| | --- | | |
| D,A | DE-A-3 643 815 (IZADPANAH) | | |
| | ----- | | |

|  |  |
|---|---|
|  | **RECHERCHIERTE SACHGEBIETE (Int. Cl.5)** <br><br> A 61 F |

Der vorliegende Recherchenbericht wurde für alle Patentansprüche erstellt

| Recherchenort | Abschlußdatum der Recherche | Prüfer |
|---|---|---|
| DEN HAAG | 06-12-1989 | STEENBAKKER J. |